# FASCICULE DE BREVET EUROPEEN

(11) **EP 3 650 551 B1**
(45) Date de publication et mention de la délivrance du brevet: **23.06.2021**
(21) Numéro de dépôt: 19207915.0
(22) Date de dépôt: 08.11.2019
(51) Int. Cl.: C12Q 1/04, C12Q 1/18, G01N 33/50, G01N 33/58, G01N 15/14

(54) **PROCÉDÉ D'ISOLEMENT ET D'ANALYSE DE MICROORGANISMES CONTENUS DANS UN ÉCHANTILLON**
ISOLIER- UND ANALYSEVERFAHREN VON IN EINER PROBE ENTHALTENEN MIKROORGANISMEN
METHOD FOR ISOLATING AND ANALYSING MICROORGANISMS CONTAINED IN A SAMPLE

(30) Priorité: 12.11.2018 FR 1860440
(43) Date de publication de la demande: 13.05.2020
(73) Titulaire: MilliDrop Instruments SAS, 94160 Saint-Mandé (FR)
(72) Inventeur: BOITARD, Laurent, 95100 Argenteuil (FR); BRAMBILLA, Elisa, 75015 Paris (FR); COTTINET, Denis, 92120 Montrouge (FR)
(74) Mandataire: Lavoix

(56) Documents cités:
- US-B1- 9 851 345
- JAMES Q. BOEDICKER ET AL: "Detecting bacteria and determining their susceptibility to antibiotics by stochastic confinement in nanoliter droplets using plug-based microfluidics", LAB ON A CHIP, vol. 8, no. 8, 1 janvier 2008 (2008-01-01) , pages 1265-1272, XP055367144, ISSN: 1473-0197, DOI: 10.1039/b804911d
- DONG-KU KANG ET AL: "Rapid detection of single bacteria in unprocessed blood using Integrated Comprehensive Droplet Digital Detection", NATURE COMMUNICATIONS, vol. 5, 13 novembre 2014 (2014-11-13), page 5427, XP055367108, DOI: 10.1038/ncomms6427
- KAUSHIK ANIRUDDHA M ET AL: "Accelerating bacterial growth detection and antimicrobial susceptibility assessment in integrated picoliter droplet platform", BIOSENSORS AND BIOELECTRONICS, ELSEVIER SCIENCE LTD. UK, AMSTERDAM, NL, vol. 97, 27 juin 2017 (2017-06-27), pages 260-266, XP085112957, ISSN: 0956-5663, DOI: 10.1016/J.BIOS.2017.06.006
- ZHANG PENGFEI ET AL: "Spatially encoded picoliter droplet groups for high-throughput combinatorial analysis", 2017 19TH INTERNATIONAL CONFERENCE ON SOLID-STATE SENSORS, ACTUATORS AND MICROSYSTEMS (TRANSDUCERS), IEEE, 18 juin 2017 (2017-06-18), pages 1797-1800, XP033131078, DOI: 10.1109/TRANSDUCERS.2017.7994418
- MUHAMMAD ASIM FARIDI ET AL: "Elasto-inertial microfluidics for bacteria separation from whole blood for sepsis diagnostics", JOURNAL OF NANOBIOTECHNOLOGY, vol. 15, no. 1, 4 janvier 2017 (2017-01-04), XP055612303, DOI: 10.1186/s12951-016-0235-4
- LAURENT BOITARD ET AL: "Growing microbes in millifluidic droplets", ENGINEERING IN LIFE SCIENCES, vol. 15, no. 3, 10 mars 2015 (2015-03-10), pages 318-326, XP055228406, DE ISSN: 1618-0240, DOI: 10.1002/elsc.201400089
- LARYSA BARABAN ET AL: "Millifluidic droplet analyser for microbiology", LAB ON A CHIP, vol. 11, no. 23, 1 janvier 2011 (2011-01-01), page 4057, XP055080174, ISSN: 1473-0197, DOI: 10.1039/c1lc20545e

## Description

La présente invention concerne un procédé d'isolement et d'analyse de microorganismes contenus dans un échantillon.

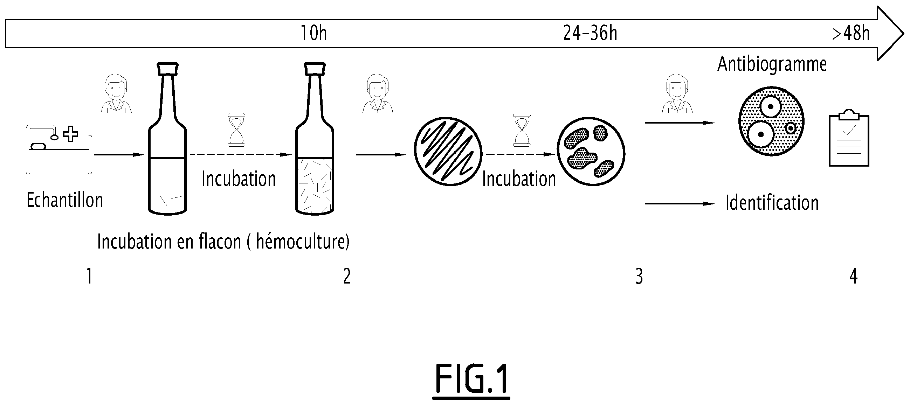

Pour l'étude de paramètres fonctionnels de microorganismes, tels qu'une sensibilité à un antibiotique, un métabolisme particulier, la production de toxines ou encore une activité enzymatique, il est connu de prélever un échantillon, par exemple un échantillon sanguin de 10 mL et de le diluer dans un flacon d'hémoculture de 40 mL tel que représenté sur la Figure 1. Le prélèvement est, par exemple, effectué en laboratoire. Lorsque l'échantillon provient d'un patient chez lequel une infection par un microorganisme pathogène, typiquement une bactérie, est suspectée, un premier traitement antibiotique est généralement indiqué au patient en attente d'une confirmation ou d'une infirmation de la nature du microorganisme pathogène suspecté et de sa sensibilité à un antibiotique.

Dans cet exemple, l'échantillon sanguin dilué est placé en incubation, typiquement à une température de 37°C pendant une durée de 6 heures à 5 jours jusqu'à détection de croissance de microorganismes. Si une croissance est détectée, l'hémoculture est dite positive. L'hémoculture est détectée positive en moyenne 24h après le lancement de l'incubation. Les hémocultures positives sont reprises pour ensemencer des boîtes de Pétri comprenant une gélose pour isoler les microorganismes. La reprise des hémocultures positives dépend de la présence et la disponibilité du personnel technique compétent, ce qui peut conduire à des délais supplémentaires pouvant aller jusqu'à 24h. Les flacons positifs sont traités plus ou moins rapidement selon la disponibilité du personnel au laboratoire et selon les horaires d'ouverture du laboratoire.

Après 24 heures d'incubation supplémentaire à 37°C, le microorganisme est isolé sous forme de colonies individualisées sur une gélose.

Ces colonies sont utilisées pour réaliser une identification de l'agent infectieux ainsi que des tests de sensibilité aux antibiotiques dénommés « antibiogrammes ».

L'identification nécessite une durée comprise entre 1h pour l'analyse en spectrométrie de masse et 24h pour les méthodes culturales. Les tests de sensibilité nécessitent une durée comprise entre 6 et 24 heures pour obtenir des résultats.

Selon les résultats, il est proposé un ajustement du traitement antibiotique, soit 48 à 72 heures après la réception de l'échantillon. Les délais d'attente des résultats peuvent être très variables car les opérateurs ne sont pas toujours disponibles pour prendre en charge les échantillons entre les différentes étapes, notamment en fonction des horaires de travail du laboratoire.

Un tel procédé est donc long et fastidieux à mettre en œuvre, les contaminations peuvent survenir au cours de plusieurs étapes du fait de nombreuses manipulations par des opérateurs. Ces contaminations peuvent ainsi fausser les résultats de l'identification et des tests de sensibilité à des antibiotiques. L'art antérieur propose différents procédés pour l'analyse de microorganismes. Ainsi, Boedicker et al. (Lab On A Chip, volume 8, 2008), Kang et al., (Nature communications, volume 5, 2014), Aniruddha et al. (Biosensors and Bioelectronics, volume 97, 2017) et Pengfei et al. (2017, 19TH INTERNATIONAL CONFERENCE ON SOLID-STATE SENSORS, ACTUATORS AND MICROSYSTEMS (TRANSDUCERS)) proposent différents procédés pour l'analyse de microorganisme à l'aide de dispositifs de type microfluidique.

Un but de l'invention est de fournir un procédé réduisant significativement la durée d'isolement et d'analyse de microorganismes, et limitant la survenue de contaminations.

A cet effet, l'invention a pour objet un procédé d'isolement et d'analyse de microorganismes contenus dans un échantillon comprenant les étapes suivantes :
- prélèvement d'un volume déterminé dans un échantillon, le volume déterminé représentant tout ou partie de cet échantillon, susceptible de contenir au moins un microorganisme,
- fragmentation du volume prélevé en une pluralité de compartiments comprenant un milieu de culture, le volume de chaque compartiment étant inférieur à 10 µL, chaque compartiment étant isolé des autres compartiments et dépourvu d'interface avec l'atmosphère ambiante,
- incubation de l'au moins un microorganisme dans les compartiments pendant une durée déterminée,
- avant, ou pendant l'incubation, détection des compartiments comprenant au moins un microorganisme,
- prolongation de l'incubation après la détection des compartiments comprenant au moins un microorganisme jusqu'à détecter une quantité de microorganisme définie,
- récupération du contenu des compartiments détectés comprenant au moins un microorganisme dans des réceptacles pour un usage ultérieur,
- détermination d'au moins un paramètre fonctionnel relatif à un microorganisme dans les compartiments comprenant au moins un microorganisme,
dans lequel les étapes de fragmentation, d'incubation, de détection et de détermination d'au moins un paramètre fonctionnel sont intégrées dans un système automatisé d'isolement et d'analyse de microorganismes.

Le procédé d'isolement et d'analyse de microorganismes selon l'invention peut comprendre l'une ou plusieurs des caractéristiques suivantes, prises isolément ou selon toute combinaison techniquement possible :
- le contenu des compartiments, dans lesquels au moins un microorganisme a été détecté, est récupéré dans des réceptacles tandis que les autres compartiments sont éliminés ;
- l'échantillon est un échantillon de sang ;
- chaque compartiment est une goutte ;
- le paramètre fonctionnel relatif à un microorganisme est une sensibilité à un antibiotique ;
- le procédé comprend en outre, après l'étape de détection, une étape de récupération d'au moins une partie des compartiments comprenant au moins un microorganisme hors du système automatisé d'isolement et d'analyse de microorganismes ;
- le procédé comprend en outre une étape de mesure d'un paramètre indicatif de la croissance d'un microorganisme dans les compartiments à différents instants ;
- la durée de l'étape d'incubation de l'au moins un microorganisme dans les compartiments est supérieure à 1 heure ;
- le procédé comprend en outre une étape de préparation des compartiments pour la détermination de l'au moins un paramètre fonctionnel ;
- après prolongation de l'incubation, les compartiments comprenant au moins un microorganisme détecté contiennent en cumulé au moins 10³ microorganismes ;
- la durée de la prolongation de l'incubation est au moins égale à 30 minutes.

L'invention a également pour objet un système automatisé d'isolement et d'analyse de microorganismes contenus dans un échantillon, le système comprenant :
- un dispositif de fragmentation d'un volume déterminé d'un échantillon susceptible de contenir au moins un microorganisme en une pluralité de compartiments dont le volume est inférieur à 10 µL, chaque compartiment étant isolé des autres compartiments et dépourvu d'interface avec l'atmosphère ambiante,
- un dispositif d'incubation de l'au moins un microorganisme dans les compartiments pendant une durée déterminée,
- un dispositif de détection des compartiments comprenant au moins un microorganisme, et
- un dispositif de détermination d'au moins un paramètre fonctionnel, propre à déterminer au moins un paramètre fonctionnel relatif à un microorganisme dans les compartiments comprenant au moins un microorganisme,
caractérisé par une unité centrale de commande propre à piloter le dispositif d'incubation et le dispositif de détection pour réaliser :
- une incubation de l'au moins un microorganisme dans les compartiments pendant une durée déterminée,
- une détection des compartiments comprenant au moins un microorganisme avant ou après l'incubation, et
- une prolongation de l'incubation.

Le système automatisé d'isolement et d'analyse de microorganismes selon l'invention peut comprendre l'une ou plusieurs des caractéristiques suivantes, prises isolément ou suivant toute combinaison techniquement possible :
- le système contient dans le dispositif d'incubation, dans le dispositif de détection ou dans le dispositif de détermination d'au moins un paramètre fonctionnel, une pluralité de compartiments dont le volume est inférieur à 10 µL ;
- le dispositif de détermination d'au moins un paramètre fonctionnel, propre à déterminer au moins un paramètre fonctionnel relatif à un microorganisme dans les compartiments comprenant au moins un microorganisme, est propre à mettre en oeuvre un test de sensibilité à au moins un antibiotique dans les compartiments ;
- le système comprend:
- un tube,
- un module de génération d'un train de gouttes ordonnées dans un fluide porteur, chaque compartiment correspondant à une goutte, le module de génération d'un train de gouttes comprenant le dispositif de fragmentation, et
- un dispositif de mise en circulation du train de gouttes dans le tube, propre à mettre en circulation le train de gouttes entre le dispositif de fragmentation, le dispositif d'incubation, le dispositif de détection et le dispositif de détermination d'au moins un paramètre fonctionnel ;
- le dispositif d'incubation comprend au moins un dispositif de stockage des gouttes propre à être régulé en température.

L'invention sera mieux comprise à la lecture de la description qui va suivre, donnée uniquement à titre d'exemple et faite en se référant aux dessins annexés, sur lesquels :
- la figure 1 est une représentation schématique d'un procédé d'isolement et d'analyse de microorganismes contenus dans un échantillon selon un état de la technique connu ;
- la figure 2 est une représentation schématique d'un système d'isolement et d'analyse de microorganismes selon l'invention ;
- la figure 3 est une vue analogue à la figure 1 d'un procédé selon l'invention ;
- la figure 4 est une courbe représentant la détection de gouttelettes contenant des bactéries en croissance en fonction du temps à l'aide d'un marquage fluorescent ; et
- la figure 5 est une courbe représentant la croissance de la souche K12 MC4100 d'Escherichia coli en présence de différentes concentrations d'ampicilline.

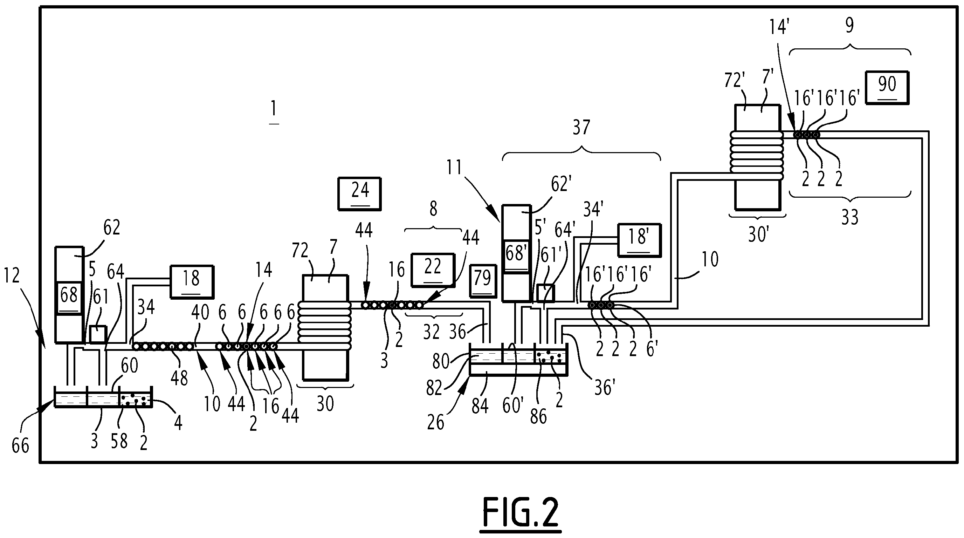

La figure 2 illustre un exemple de système automatisé d'isolement et d'analyse de microorganismes 1.

Le système automatisé d'isolement et d'analyse de microorganismes 1 est un système pour isoler des microorganismes 2 contenus dans un échantillon 4 susceptible de contenir au moins un microorganisme et analyser au moins un paramètre fonctionnel des microorganismes 2. Par exemple, l'échantillon 4 contient une seule espèce de microorganisme 2. En variante, l'échantillon 4 contient plusieurs espèces différentes de microorganismes 2.

Les microorganismes 2 sont, par exemple, des bactéries, des levures, des algues ou des champignons filamenteux. Les microorganismes 2, sont, par exemple, des cellules procaryotes ou eucaryotes.

Les microorganismes 2 sont, par exemple, des microorganismes pathogènes.

L'échantillon 4 est, par exemple, un échantillon de sang. De préférence, l'échantillon 4 est un échantillon de sang humain.

En variante, l'échantillon 4 est un échantillon d'urine, de liquide céphalorachidien, de liquide broncho-alvéolaire, de fèces, de prélèvement de peau ou de prélèvement intestinal. Selon cette variante, l'échantillon est un échantillon humain ou animal.

Selon une autre variante, l'échantillon 4 est un échantillon de sol, de nourriture, de boisson, d'eau de baignade, ou de produit industriel.

Avantageusement, l'échantillon 4 comprend un milieu de culture 3.

Le milieu de culture 3 comprend un milieu de culture liquide adapté à la survie et à la croissance des microorganismes 2 comme une solution tamponnée complétée avec des nutriments de culture, des vitamines, des donneurs et des accepteurs d'électrons. Par exemple, le milieu de culture 3 liquide est du bouillon Trypticase soja, du bouillon Wilkins Chalgren, du *Yeast extract-Casein hydrolysate-Fatty Acids* (YCFA), du *Cooked Meat Broth* (CMB), du *Chopped Meat Carbohydrate Broth,* du *Columbia broth,* du milieu de culture au sang, du milieu infusion cœur-cerveau, (*Brain-heart infusion medium* BHIM), du milieu à base d'extrait d'estomac de mouton, du Bouillon Mueller-Hinton (MH), du Bouillon Mueller-Hinton pour bactéries fastidieuses (MH-F), ou du bouillon Wilkins Chalgren.

Avantageusement, le milieu de culture 3 comprend des activateurs de croissance microbienne. Par exemple, le milieu de culture 3 comprend du facteur X (hémine) et du facteur V (nicotinamide-adénine-dinucléotide, NAD), et de la vitamine B6.

Avantageusement le milieu de culture 3 comprend des résines actives, échangeuses d'ions, pour l'adsorption de molécules inhibitrices de la croissance de certains microorganismes.

Le système automatisé d'isolement et d'analyse de microorganismes 1, selon l'invention, comprend un dispositif de fragmentation 5 d'un volume déterminé d'un échantillon susceptible de contenir au moins un microorganisme 2 en une pluralité de compartiments 6 dont le volume est inférieur à 10 µL, chaque compartiment 6 étant isolé des autres compartiments 6 et dépourvu d'interface avec l'atmosphère ambiante, un dispositif d'incubation 7 du ou des microorganismes 2 dans les compartiments 6 pendant une durée déterminée, un dispositif de détection 8 des compartiments 6 comprenant au moins un microorganisme 2, et un dispositif de détermination d'au moins un paramètre fonctionnel 9, propre à déterminer au moins un paramètre fonctionnel relatif à un microorganisme 2 dans les compartiments 6 comprenant au moins un microorganisme 2.

Avantageusement, le système 1 comprend, en outre, un dispositif de préparation 11 des compartiments 6 pour la détermination d'au moins un paramètre fonctionnel.

Avantageusement, le système 1 comprend une enceinte intégrant l'ensemble des dispositifs 5, 7, 8, 11, 9 qui le composent.

Avantageusement, au moins l'un parmi le dispositif d'incubation 7, le dispositif de détection 8 et le dispositif de détermination d'au moins un paramètre fonctionnel 9 contient des compartiments 6.

Dans le système automatisé d'isolement de microorganismes 1 représenté sur la figure 2, chaque compartiment 6 est une goutte 16, 16'.

Le système automatisé d'isolement et d'analyse de microorganismes 1 comporte un tube 10, un module de génération 12 d'un train 14 de gouttes 16 destiné à circuler dans le tube 10, et un dispositif de mise en circulation 18 du train 14 de gouttes 16 dans le tube 10. Le dispositif de préparation 11 des compartiments 6 pour la détermination d'au moins un paramètre fonctionnel est propre à générer un train 14' de gouttes 16'. Avantageusement, le système automatisé d'isolement et d'analyse de microorganismes 1 comporte également un dispositif de mise en circulation 18' du train 14' de gouttes 16' dans le tube 10. Le tube 10 connecte fluidiquement le dispositif de fragmentation 5, le dispositif d'incubation 7 et le dispositif de détection 8 d'une part, et le dispositif de préparation 11 des compartiments 6 et le dispositif de détermination d'au moins un paramètre fonctionnel 9 d'autre part.

Le système automatisé d'isolement et d'analyse de microorganismes 1 comprend également un dispositif de mesure 22 dans le dispositif de détection 8, une unité centrale 24 de commande et un dispositif de récupération 26.

Dans ce qui suit, les termes « amont » et « aval » et les termes « entrée » et « sortie » sont utilisés en référence aux sens normaux de circulation des fluides dans le système. Le terme « diamètre » désigne l'étendue maximale du tube considéré dans un plan transversal, c'est-à-dire perpendiculaire, à l'axe central du tube, par exemple, le diamètre d'un cercle dans le cas où la section transversale du tube est circulaire ou la diagonale d'un rectangle dans le cas où la section transversale du tube est rectangulaire.

Le tube 10 est un tube capillaire ou tube fluidique à l'échelle millimétrique c'est-à-dire présentant un diamètre intérieur de l'ordre du dixième de millimètre au millimètre. De préférence, le tube 10 présente un diamètre intérieur compris entre 0,2 mm et 3 mm.

Le tube 10 présente une section transversale intérieure de contour arrondi ou dépourvu d'angles. Par exemple, le tube 10 présente une section transversale circulaire ou elliptique, ou polygonale telle que rectangulaire.

Le tube 10 présente au moins une zone d'incubation 30, une zone de mesure 32 et une zone de détermination d'un paramètre fonctionnel 33. En outre, le tube 10 présente au moins une extrémité d'entrée 34 et au moins une extrémité de sortie 36.

Avantageusement, le système 1 présente en outre une zone de préparation 37 des compartiments 6' pour la détermination d'un paramètre fonctionnel.

Le module de génération 12 d'un train 14 de gouttes 16 est propre à générer un train 14 de gouttes 16.

Un train 14 de gouttes 16 est une succession de gouttes 16 ordonnées dans un fluide porteur 40.

Le fluide porteur 40 est, avantageusement, une phase organique, notamment une phase huileuse. Le fluide porteur 40 comprend, par exemple, des hydrofluoroéthers comme FC-40 ou HFE-7500, formant une huile fluorée. En variante, le fluide porteur 40 comprend une huile silicone ou une huile organique telle que de l'huile minérale.

Le fluide porteur 40 est apte à séparer deux gouttes 16 successives du train 14 de gouttes 16.

Chaque goutte 16 comprend un fluide interne 48 non miscible avec le fluide porteur 40. On entend par non miscible que le coefficient de partage entre les deux fluides est inférieur à 10⁻³. Le fluide interne 48 est, avantageusement, une phase aqueuse.

Le train 14 de gouttes 16 comprend avantageusement des séparateurs 44. Un séparateur 44 est un volume de fluide non miscible avec le fluide porteur 40 et non miscible avec le fluide interne 48, ou une bulle de gaz. Le séparateur 44 favorise l'espacement entre deux gouttes 16 successives du train 14 de gouttes 16 pour prévenir le contact ou la fusion des gouttes 16.

Dans un exemple non représenté, le train 14 de gouttes 16 comprend un séparateur 44 entre chaque goutte 16.

Chaque goutte 16 du train 14 de gouttes 16 constitue un compartiment 6 isolé des autres compartiments 6 et dépourvu d'interface avec l'atmosphère ambiante.

Chaque goutte 16 est une goutte de culture formée à partir de l'échantillon 4.

Le volume de chaque goutte 16 du train 14 de gouttes 16 est inférieur à 10 µL. De préférence, le volume de chaque goutte 16 du train 14 de gouttes 16 est inférieur à 5 µL. De préférence, le volume des gouttes 16 du train 14 de gouttes 16 est compris entre 0,2 µL et 2 µL, de préférence encore entre 0,6 µL et 1,2 µL. De préférence encore, le volume des gouttes 16 du train 14 de gouttes 16 est sensiblement égal à 1 µL.

Dans un exemple, le volume des gouttes 16 du train 14 de gouttes 16 est sensiblement le même d'une goutte 16 à l'autre.

Le volume des gouttes est étudié pour que chaque goutte 16 puisse contenir une quantité suffisante de microorganismes 2 nécessaire pour mettre en oeuvre une étape de détermination d'un paramètre fonctionnel relatif à un microorganisme 2.

Les gouttes 16 comprennent du milieu de culture 3.

De préférence, au moins une goutte 16 comprend un microorganisme 2.

En variante, les gouttes 16 comprennent, en outre, un indicateur de croissance. Par exemple, l'indicateur est un rapporteur fluorescent ou un rapporteur chromogénique d'activité métabolique. Le rapporteur fluorescent est par exemple carboxyfluorescein diacetate (CFDA) ou la résazurine.

L'indicateur de croissance est par exemple un rapporteur d'activité métabolique luminescent ou bioluminescent comme indicateur de la présence d'ATP utilisant une luciférase.

La goutte 16 comprend des entités de signalisation propres à indiquer la présence dans la goutte 16 d'une enzyme ou d'autres biomolécules, par exemple des glycopeptides. Par exemple, les entités de signalisation sont des substrats fluorogéniques qui indiquent la présence d'une enzyme car ils sont modifiés par cette enzyme. Par exemple, les entités de signalisation sont des substrats fluorogéniques qui réagissent avec des biomolécules des membranes cellulaires. Par exemple les substrats fluorogéniques sont liés à des lectines ou encore à un composé qui comprend une zinc(II)dipicolylamine.

La goutte 16 comprend en outre des éléments sécrétés par le microorganisme 2 comme des protéines ou des molécules. Ces substances sécrétées sont susceptibles de modifier le potentiel hydrogène (pH), l'impédance, ou la composition en gaz dissous de la goutte 16. Par exemple, la goutte 16 comprend des entités de signalisation propres à indiquer la présence d'éléments sécrétés et à permettre leur quantification. Par exemple, les entités de signalisation sont des substrats fluorogéniques qui indiquent la présence d'une enzyme. Par exemple, les entités de signalisation sont des substrats fluorogéniques qui indiquent la variation du pH en solution.

Le module de génération 12 du train 14 de gouttes 16 comporte par exemple un ou une pluralité de réservoirs d'échantillon 58, un ou une pluralité de réservoirs 60 de fluides nécessaires à la formation du train 14 de gouttes 16, un dispositif de prélèvement 62 et un circuit d'entrée 64.

Le module de génération 12 comporte, en outre, un réservoir complémentaire 61. Le réservoir complémentaire 61 comporte du fluide porteur 40.

Par exemple, des réservoirs d'échantillon 58, ou des réservoirs 60 de fluides nécessaires à la formation du train 14 de gouttes 16 sont différents compartiments d'une plaque 66 de microtitration. En variante, les réservoirs 58, 60 sont des tubes à essais tel que des tubes Falcon® ou des microtubes comme ceux commercialisés par Eppendorf®. En variante, les réservoirs 58, 60 sont des tubes de prélèvement de sang ou des flacons d'hémoculture.

Un réservoir d'échantillon 58 comprend l'échantillon 4. Par exemple, d'autres réservoirs de microorganismes 58 contiennent d'autres échantillons 4.

Par exemple, un réservoir 60 comprend le milieu de culture 3.

Par exemple, d'autres réservoirs 60 contiennent des réactifs à mettre dans la goutte 16 ou du fluide porteur 40 pour préparer les gouttes 16. Par exemple, d'autres réservoirs 60 contiennent des solutions tampon, des entités de signalisation ou des additifs pour favoriser la croissance des microorganismes 2.

Le dispositif de prélèvement 62 est propre à prélever des solutions dans chacun des réservoirs 58, 60 de sorte à former un train 14 de gouttes 16 ordonnées dans le fluide porteur 40.

Par exemple, au moins deux réservoirs d'échantillon 58 comprennent chacun un échantillon 4 distinct. Le train 14 de gouttes 16 ordonnées contient une première série de gouttes 16 issues du premier réservoir d'échantillon 58 et une deuxième série de gouttes 16 distincte de la première série de gouttes 16 issues du deuxième réservoir de microorganismes 58.

Le dispositif de prélèvement 62 est propre à préparer le train de gouttes dans le circuit d'entrée 64.

Par exemple, le dispositif de prélèvement 62 comporte un bras robotisé de pipetage. En variante ou en complément, le dispositif de prélèvement 62 comporte une tête d'aspiration. L'utilisation d'un dispositif de prélèvement 62 robotisé permet de limiter l'espace nécessaire aux manipulations.

Par exemple le dispositif de prélèvement 62 comprend un réservoir de gaz 68. Le réservoir de gaz 68 sert, par exemple, à mettre sous pression les différents réservoirs 60, 61 pour faciliter le prélèvement. Par exemple, le dispositif de prélèvement 62 est propre à injecter un fluide dans le circuit d'entrée 64 en poussant le fluide depuis le réservoir 60, 61 au moyen du gaz jusque dans le circuit d'entrée 64.

En variante ou en complément, le dispositif de prélèvement 62 comporte une pompe d'aspiration placée en dérivation sur le tube 10. Par exemple, la pompe est placée à la sortie 36. Selon un autre exemple, la pompe est placée en aval du dispositif de détection 8. La pompe est propre à aspirer les différents fluides et à placer les réservoirs 60, 61 sous dépression. Par exemple, la pompe est un compresseur ou une pompe gerotor.

Le dispositif de prélèvement 62 comprend avantageusement un robot propre à préparer le contenu des réservoirs 60 dans l'enceinte du système.

Le circuit d'entrée 64 est connecté à l'entrée 34 du tube 10. Le circuit d'entrée 64 comporte le dispositif de fragmentation 5 propre à générer des gouttes 16 à partir de l'échantillon 4 prélevé et d'un fluide porteur 40.

Par exemple, le dispositif de fragmentation 5 consiste en un décrochement ou une marche facilitant la fragmentation des fluides et la génération des gouttes 16. En variante, le dispositif de fragmentation 5 consiste en une jonction à focalisation de flux, dite jonction « flow focusing », ou une jonction T.

Le fluide porteur 40 est, par exemple, injecté, au niveau du circuit d'entrée 64, le long du tube 10 par le dispositif d'injection de sorte à former les gouttes 16 du train 14 de gouttes 16 par co-écoulement.

Le dispositif de mise en circulation 18 du train 14 de gouttes 16 est propre à déplacer le train 14 de gouttes 16 au sein du tube 10 depuis l'entrée 34 jusqu'à la sortie 36.

Le dispositif de mise en circulation 18 comprend, par exemple, une unité de soufflage et/ou une unité d'aspiration.

Le dispositif de mise en circulation 18 est avantageusement propre à faire circuler le train 14 de gouttes 16 de la zone d'incubation 30 à la zone de mesure 32 puis de la zone de mesure 32 à la zone d'incubation 30. Les gouttes 16 peuvent ainsi être déplacées dans les deux sens dans le tube 10.

Par exemple, la zone de mesure 32 est située en aval de la zone d'incubation 30. Le dispositif de mise en circulation 18 est propre à faire passer les gouttes 16 de la zone d'incubation 30 à la zone de mesure 32 pour détecter les gouttes 16 comprenant au moins un microorganisme 2 et mesurer le paramètre indicatif du contenu des gouttes 16. Le dispositif de mise en circulation 18 est également propre à faire passer les gouttes 16 de la zone de mesure 32 à la zone d'incubation 30 pour poursuivre l'incubation de la goutte 16.

Le dispositif de mise en circulation 18 est propre à générer un débit du train 14 de gouttes 16 et de fluide porteur 40 dans le tube compris entre 0,1 mL/h et 100 mL/h.

Le dispositif d'incubation 7 est un dispositif d'incubation du train 14 de gouttes 16 dans le tube 10.

Le dispositif d'incubation 7 est propre à contrôler la température de la zone d'incubation 30 du tube 10. Par exemple, le dispositif d'incubation 7 est propre à chauffer ou à refroidir la zone d'incubation 30 du tube à une température comprise entre 4°C et 100°C, par exemple comprise entre 20 °C et 50°C et notamment à 37°C. Dans un exemple, pour isoler et analyser des microorganismes associés à des pathologies humaines, la température est réglée à 37°C.

Le dispositif d'incubation 7 comprend un dispositif de stockage des gouttes 16 propre à être régulé en température. Le dispositif de stockage des gouttes 16 est par exemple une bobine 72, la partie du tube 10 correspondant à la zone d'incubation 30 étant enroulée pour former une bobine 72. Cet enroulement permet de réduire l'encombrement nécessaire pour avoir une grande longueur d'incubation.

Avantageusement, le dispositif d'incubation 7 comprend une pluralité de bobines 72. Ceci permet d'effectuer plusieurs incubations en parallèle, ou d'effectuer l'incubation d'un grand nombre de gouttes. Ceci est particulièrement avantageux pour traiter plusieurs échantillons 4 en même temps. Une bobine supplémentaire permet de stocker les gouttes 16 en cours d'incubation.

Avantageusement, lorsque le nombre de bobines 72 est supérieur à 2, plusieurs dispositifs de détection 8 et plusieurs modules de génération 12 sont utilisés et chacun est utilisé avec au moins une bobine 72.

Par exemple la longueur totale de tube 10 enroulée dans la zone d'incubation 30 est comprise entre 5 mètres et 1000 mètres. Par exemple chaque bobine 72 contient une longueur de tube 10 comprise entre 5 et 50 mètres.

En variante, le dispositif d'incubation comprend une chambre délimitée par des parois d'isolation thermique, un élément chauffant et refroidissant tel qu'un module Peltier, un ventilateur permettant de faire circuler le gaz de convection de contenu et une sonde de température.

Le dispositif de détection 8 des gouttes 16 comprenant au moins un microorganisme 2 est par exemple disposé en aval de la zone d'incubation 30.

Le dispositif de mesure 22 est propre à mesurer un paramètre indicatif du contenu des gouttes 16 dans le tube 10 au niveau de la zone de mesure 32 à différents instants.

Par exemple, le paramètre est représentatif de la croissance d'un microorganisme dans la goutte 16 ou de la survie des cellules. De telles mesures successives permettent à la fois de détecter les gouttes 16 comprenant au moins un microorganisme 2, présentant une activité métabolique ou en division, et de réaliser pour chaque goutte 16 des courbes de cinétiques de croissance au sein de la goutte 16 au cours de l'incubation.

Par exemple, le dispositif de mesure 22 est propre à effectuer une mesure de densité optique, de diffusion de la lumière, et/ou une analyse sur une image d'une goutte.

En variante ou en complément le dispositif de mesure 22 est propre à détecter ou à quantifier une protéine.

Par exemple, la mesure est une mesure optique, comme une mesure de fluorescence, une mesure de diffusion de la lumière, une analyse d'image, une mesure de spectroscopie Raman ou de spectroscopie Infra-Rouge. La mesure de fluorescence peut consister à mesurer l'autofluorescence des microorganismes.

Avantageusement, le dispositif de mesure 22 est propre à mettre en oeuvre un étalonnage pour vérifier la qualité des mesures effectuées.

L'unité centrale 24 comporte une mémoire et un microprocesseur. L'unité centrale 24 est propre à enregistrer les données du dispositif de mesure 22 pour chaque goutte 16.

L'unité centrale 24 est propre à analyser les mesures effectuées pour une goutte 16 et à contrôler la mise en circulation de la goutte 16 vers la zone de préparation 37 pour la détermination d'un paramètre fonctionnel, la poursuite de l'incubation, la récupération de la goutte 16 ou l'évacuation de la goutte 16 selon le résultat de l'analyse.

Avantageusement, l'unité centrale 24 est propre à déterminer si un microorganisme 2 est détecté dans une goutte 16 d'après les mesures du dispositif de mesure 22. L'unité centrale 24 est propre à avertir un utilisateur ou le système d'information du laboratoire qu'un microorganisme 2 a été détecté dans au moins une goutte 16, ou à transmettre le nombre de gouttes 16 dans lesquelles un microorganisme 2 a été détecté.

Avantageusement, l'unité centrale 24 est propre à décider de prolonger l'incubation des gouttes 16 dans la zone d'incubation 30 pour garantir que la quantité de microorganismes 2 produits dans les gouttes 16 est suffisante en vue des préparations et mesures planifiées dans le système 1 ou en dehors du système 1.

Avantageusement encore, l'unité centrale 24 est propre à ajuster la prolongation de l'incubation en fonction de paramètres quantitatifs calculés à partir des mesures effectuées sur la goutte 16 par le dispositif de mesure 22. Des exemples de paramètres quantitatifs seront décrits ci-après.

L'unité centrale 24 est propre à déterminer l'appartenance du microorganisme 2 présent dans la goutte 16, à un groupe de microorganismes d'après des paramètres quantitatifs calculés à partir des mesures effectuées sur la goutte 16. Par exemple les paramètres quantitatifs peuvent permettre de déterminer si le microorganismes 2 est Gram positif ou Gram négatif, ou encore s'il s'agit d'une bactérie ou d'un champignon. Par exemple, l'unité centrale 24 est propre à utiliser les paramètres quantitatifs pour définir des groupes ad hoc, c'est-à-dire réaliser un partitionnement de données, et en déduire une évaluation de probabilité que les microorganismes 2 détectés dans les gouttes 16 appartiennent à une pluralité d'espèces. L'unité centrale 24 est propre à avertir l'utilisateur ou le système d'information du laboratoire cette probabilité de détection d'une variété de microorganismes 2, ce qui correspond à un échantillon couramment désigné comme polymicrobien.

Avantageusement, le système 1 comprend en aval du dispositif de mesure 22 un dispositif 79 d'addition d'au moins un réactif. Le dispositif 79 comprend par exemple une pluralité de réservoirs propres à contenir chacun un réactif. Le dispositif 79 est propre à introduire le réactif dans une goutte 16 dans laquelle au moins un microorganisme 2 a été détecté.

Le réactif comprend par exemple, de la matrice pour la spectrométrie de masse MALDI, des enzymes, de l'acide désoxyribonucléique (ADN) ou de l'acide ribonucléique (ARN).

Le dispositif de récupération 26 comprend, par exemple, au moins un récipient de récupération 80 comprenant plusieurs réceptacles 82, et un dispositif de déplacement 84 du récipient de récupération 80 par rapport à la sortie 36 du tube 10 de sorte que chaque goutte 16 à récupérer soit transférée vers un réceptacle 82. Les gouttes 16 à évacuer sont par exemple transférées vers un réceptacle 82 différent dédié ou un autre récipient de récupération 80. Par exemple, les gouttes 16 à récupérer sont transférées chacune vers un réceptacle 82 différent. Dans un autre exemple, l'unité centrale 24 est propre à déterminer pour chaque goutte 16 à récupérer son appartenance à un groupe d'après les paramètres quantitatifs calculés à partir des mesures effectuées sur la goutte 16, et les gouttes appartenant à un même groupe sont toutes transférées dans un seul réceptacle 82 mais un réceptacle 82 différent pour chaque groupe.

Les gouttes 16 récupérées sont par exemple utilisées pour une caractérisation hors du système 1. La caractérisation est, par exemple, une identification des microorganismes présents ou une caractérisation Gram+/Gram-. Par exemple, les microorganismes 2 sont caractérisés par spectrométrie de masse MALDI-TOF, ou par l'utilisation de galeries API.

Avantageusement, le dispositif de récupération 26 comprend un support adapté à la technique MALDI-TOF pour l'identification des espèces microbiennes.

Un tel support est propre à être transféré automatiquement par un convoyeur dans un spectromètre de masse adapté pour réaliser la caractérisation.

Le dispositif de préparation 11 des compartiments 6 pour la détermination d'au moins un paramètre fonctionnel est propre à préparer de nouveaux compartiments 6' comprenant des microorganismes 2 issus des gouttes 16 récupérées et pour lesquelles on souhaite déterminer un paramètre fonctionnel après avoir préparé plusieurs compartiments 6'.

Le dispositif de préparation 11 des compartiments 6' est propre à générer un train 14' de gouttes 16' et comprend avantageusement les mêmes éléments que le module de génération 12 d'un train 14 de gouttes 16.

Le dispositif de préparation 11 des compartiments 6' comporte par exemple un ou une pluralité de réservoirs de microorganismes 86, un ou une pluralité de réservoirs 60' de fluides nécessaires à la formation du train 14' de gouttes 16', un dispositif de transfert des microorganismes 2 issus des gouttes 16 dans les réceptacles 82 vers les réservoirs de microorganismes 86,un dispositif de prélèvement 62' et un circuit d'entrée 64'.

Le dispositif de préparation 11 des compartiments 6' comporte, en outre, un réservoir complémentaire 61'. Le réservoir complémentaire 61' comporte du fluide porteur 40.

Le dispositif de préparation 11 des compartiments 6' comporte, en outre, une pluralité de réservoirs d'antibiotiques et un dispositif d'inoculation propre à inoculer un antibiotique issu d'un réservoir dans les gouttes 16'. Par exemple, certains réservoirs d'antibiotiques ne comprennent pas d'antibiotique.

Par exemple le dispositif de prélèvement 62 comprend un réservoir de gaz 68'.

Le circuit d'entrée 64' est connecté à l'entrée 34' du tube 10. Le circuit d'entrée 64' comporte un dispositif de fragmentation 5' propre à générer des gouttes 16' à partir des microorganismes 2 issus des gouttes 16 récupérées et du fluide porteur 40.

Par exemple, le dispositif de fragmentation 5' consiste en un décrochement ou une marche facilitant la fragmentation des fluides et la génération des gouttes 16'. En variante, le dispositif de fragmentation 5' consiste en une jonction à focalisation de flux, dite jonction « flow focusing », ou une jonction T.

Le fluide porteur 40 est, par exemple, injecté, au niveau du circuit d'entrée 64', le long du tube 10 par le dispositif d'injection de sorte à former les gouttes 16' du train 14' de gouttes 16' par co-écoulement.

Un dispositif de mise en circulation 18' du train 14' de gouttes 16' est propre à déplacer le train 14' de gouttes 16' au sein du tube 10 depuis l'entrée 34' jusqu'à la sortie 36'.

Avantageusement les nouveaux compartiments 6' sont préparés avec des substances chimiques utiles pour la détermination d'au moins un paramètre fonctionnel (antibiotiques, sondes fluorescentes...). De préférence, le dispositif de préparation 11 des compartiments 6' est propre à préparer des concentrations différentes et/ou des natures de substance chimiques différentes.

Avantageusement, les nouveaux compartiments 6' comprennent tous des microorganismes 2 et sont de nouvelles gouttes 16' destinées à être mises en circulation vers la zone de détermination d'un paramètre fonctionnel 33.

Avantageusement, la quantité de microorganismes 2 dans les nouvelles gouttes 16' est la même pour toutes les gouttes. La quantité de microorganismes 2 est ajustée à une valeur comprise entre 10 cellules et 1000 cellules par goutte. De telles valeurs permettent de garantir la validité des résultats et leur interprétation en cohérence avec les recommandations des organismes de référence.

Par exemple, l'unité centrale 24 est propre à déterminer les étapes de dilution nécessaires d'après les paramètres quantitatifs calculés à partir des mesures effectuées sur les gouttes 16 récupérées avant leur transfert.

Par exemple, les microorganismes 2 issus des gouttes 16 récupérées dans les réceptacles 82 et utilisées pour préparer de nouveaux compartiments 6 sont resuspendus dans un ou une pluralité de réservoirs de microorganismes 86 dans un milieu de culture et dilués pour atteindre une densité de cellules contrôlée. Par exemple cette densité de cellules est contrôlée grâce à une mesure de densité optique. Avantageusement, l'unité centrale 24 est propre à enregistrer cette mesure. Avantageusement, l'unité centrale 24 est propre à déterminer les étapes de dilution nécessaires d'après la mesure de densité optique et les paramètres quantitatifs calculés à partir des mesures effectuées sur les gouttes 16 récupérées avant leur transfert.

De préférence, le dispositif d'inoculation est propre à inoculer des concentrations d'antibiotique différentes et/ou des natures d'antibiotiques différentes dans chaque goutte 16'.

Les concentrations en antibiotique dans les réservoirs comprenant un antibiotique sont par exemple comprises entre 1 µg/L et 500 mg/L.

Par exemple, l'antibiotique est l'amoxicilline, la céfoxitine, la gentamicine, la vancomycine, et/ou la pipéracilline-tazobactam.

Avantageusement, le tube 10 présente une deuxième zone d'incubation 30' disposée en aval de la zone de préparation 37 des compartiments 6 et en amont de la zone de zone de détermination d'un paramètre fonctionnel 33.

Avantageusement, la deuxième zone d'incubation 30' comporte un dispositif d'incubation 7' identique au dispositif d'incubation 7. Par exemple, le dispositif d'incubation 7' comporte une bobine 72'.

Le dispositif de mise en circulation 18' est avantageusement propre à faire circuler le train 14' de gouttes 16' de la zone de préparation 37 des compartiments 6' pour la détermination d'un paramètre fonctionnel à la deuxième zone d'incubation 30'.

Le dispositif de mise en circulation 18' est avantageusement propre à faire circuler le train 14' de gouttes 16' de la zone d'incubation 30' à la zone de détermination d'un paramètre fonctionnel 33.

Par exemple, la zone de détermination d'un paramètre fonctionnel 33 est située en aval de la deuxième zone d'incubation 30'.

Le dispositif de détermination d'au moins un paramètre fonctionnel 9 est propre à déterminer au moins un paramètre fonctionnel relatif à un microorganisme dans les compartiments comprenant au moins un microorganisme.

Par exemple, le paramètre fonctionnel est relatif à une sensibilité d'un microorganisme 2 à un antibiotique.

Le dispositif de détermination d'au moins un paramètre fonctionnel 9 comprend, par exemple, un dispositif de mesure 90 sensiblement identique au dispositif de mesure 22.

De préférence, le dispositif de mesure 90 est identique au dispositif de mesure 22. Le paramètre fonctionnel est représentatif de la croissance d'un microorganisme dans la goutte 16' en présence d'antibiotique ou de la survie des cellules en présence d'antibiotique.

L'unité centrale 24 est propre à analyser les mesures effectuées pour une goutte 16'. Par exemple si le paramètre fonctionnel est relatif à la sensibilité d'un microorganisme 2 à un antibiotique, l'unité centrale 24 est propre à déterminer une concentration minimale inhibitrice à partir des mesures sur les gouttes 16' dans le dispositif de détermination d'au moins un paramètre fonctionnel 9. Avantageusement, l'unité centrale 24 est propre à utiliser en plus optionnellement les paramètres quantitatifs calculés à partir des mesures effectuées dans le dispositif de détection 8 pour les gouttes 16 récupérées et utilisées pour la détermination d'au moins un paramètre fonctionnel 9, et optionnellement à utiliser en plus la mesure de densité optique réalisée pour contrôler la densité de cellules avant de préparer les nouvelles gouttes 16', pour déterminer la concentration minimale inhibitrice.

Par exemple, le système 1 comprend un dispositif d'affichage, propre à afficher les résultats de l'analyse des mesures. Par exemple encore, le système 1 comprend un dispositif d'impression d'un support contenant les résultats de l'analyse des mesures.

En variante non représentée, le dispositif de de préparation 11 des compartiments 6' comprend une cassette placée à une sortie du tube et pourvue d'une pluralité de réservoirs comprenant chacun du milieu de culture, un antibiotique et/ou une substance chimique utile la détermination d'au moins un paramètre fonctionnel. La cassette est pourvue d'un dispositif d'inoculation propre à inoculer une partie des gouttes récupérées dans les réservoirs. Chaque réservoir est un compartiment isolé des autres compartiments et de l'atmosphère ambiante. Par exemple, la cassette comprend entre 30 et 1000 réservoirs. Chaque réservoir est configuré pour comprendre en fin de préparation une concentration en antibiotique comprise entre 1 µg/L et 500 mg/L. Par exemple, certains réservoirs ne contiennent pas d'antibiotique.

Par exemple, les réservoirs sont recouverts par un dispositif de fermeture propre à être ouvert lors de l'inoculation de gouttes dans les réservoirs et propre à être fermé en l'absence d'inoculation de gouttes dans le réservoir. Le dispositif de fermeture comprend par exemple des électrovannes. Le dispositif de fermeture est par exemple commandé par l'unité centrale.

De préférence, au moins deux réservoirs comprennent un antibiotique différent. De préférence encore, au moins deux réservoirs comprennent des concentrations différentes d'un même antibiotique.

Selon cette variante, le dispositif de détermination d'au moins un paramètre fonctionnel comprend un dispositif de déplacement de la cassette par rapport à la sortie du tube de sorte que les gouttes à analyser soient utilisées pour inoculer les réservoirs ou les compartiments qui comprennent un antibiotique.

Avantageusement, les dimensions du système 1 sont telles qu'il puisse être disposé sur une paillasse de laboratoire. Dans un exemple, le système 1 mesure 1452 mm de longueur, 810 mm de profondeur et 993 mm de hauteur. Dans un autre exemple, le système 1 mesure 810 mm de longueur, 760 mm de profondeur et 635 mm de hauteur.

Avantageusement, le système 1 comprend un dispositif de nettoyage intégré propre à rincer, nettoyer et tester la propreté des dispositifs intégrés au système 1.Selon un mode de réalisation particulier, le système automatisé d'isolement et d'analyse de microorganismes comprend un dispositif de contrôle de l'atmosphère. Cela permet la culture de microorganismes particuliers, notamment de microorganismes anaérobies, qui meurent ou ont une cinétique de croissance plus lente lorsqu'ils sont exposés à du dioxygène à teneur atmosphérique, et peuvent utiliser la fermentation.

Le procédé d'isolement et d'analyse de microorganismes selon l'invention va maintenant être décrit en référence à la figure 2.

Le procédé comprend la fourniture d'un système automatisé 1 d'isolement et d'analyse de microorganismes.

Les réservoirs d'échantillon 58 sont remplis avec les échantillons 4 susceptibles de contenir au moins un microorganisme 2 et les réservoirs 60 avec les fluides nécessaires à la formation du train 14 de gouttes 16.

Par exemple, cette préparation est manuelle, en variante, elle est effectuée par un robot.

Le procédé comprend, en outre, le prélèvement d'un volume déterminé dans un échantillon 4.

Le dispositif de prélèvement 62 prélève des solutions dans chacun des réservoirs 58, 60 de sorte à former un train 14 de gouttes 16 ordonnées dans le fluide porteur 40.

Le volume prélevé dans l'échantillon 4 est typiquement compris entre 35 mL et 45 mL, de préférence égal à 40 mL.

Le procédé comprend, en outre, la fragmentation du volume prélevé en une pluralité de compartiments 6 comprenant un milieu de culture 3, le volume de chaque compartiment 6 étant inférieur à 10uL, chaque compartiment étant isolé des autres compartiments et dépourvu d'interface avec l'atmosphère ambiante, l'incubation de l'au moins un microorganisme 2 dans les compartiments 6 pendant une durée déterminée, avant ou pendant l'incubation, la détection des compartiments 6 comprenant au moins un microorganisme 2, la prolongation de l'incubation après la détection et la détermination d'au moins un paramètre fonctionnel relatif à un microorganisme 2 dans les compartiments 6 comprenant au moins un microorganisme 2.

Les étapes de fragmentation, de détection et de détermination du procédé sont intégrées dans le système 1 automatisé d'isolement et d'analyse de microorganismes.

Le système automatisé d'isolement et d'analyse de microorganismes est, par exemple, le système 1 de la figure 2. Selon cet exemple, le procédé comprend la génération d'un train 14 de gouttes 16 ordonnées dans un fluide porteur 40, la mise en circulation du train 14 de gouttes 16 dans le tube 10, l'incubation du train 14 de gouttes 16 dans le tube 10, et la mesure d'au moins un paramètre indicatif du contenu des gouttes 16 dans le tube 10 à différents instants au cours de l'incubation.

Le train 14 de gouttes 16 est généré par le module de génération 12 du train 14 de gouttes 16.

Dans un exemple, pour un échantillon de sang humain de 10 mL mélangé à 40 mL de milieu de culture, entre 1000 gouttes et 100000 gouttes, de préférence entre 10000 gouttes et 50000 gouttes, de préférence encore entre 39500 gouttes et 40500 gouttes sont générées pour former un ou plusieurs trains de gouttes.

Par exemple, le train 14 de gouttes 16 est mis en circulation à un débit de 10 mL/h.

La zone d'incubation 30 du tube est maintenue à une température de 37°C.

L'incubation est effectuée dans la zone d'incubation 30 du tube 10.

L'incubation est effectuée par phases successives, une mesure est effectuée à la fin de chaque phase d'incubation.

Avantageusement, chaque phase d'incubation a une durée supérieure à 1 heure.

Par exemple, au total l'incubation dure entre 1 heure et 120 heures, de préférence entre 1 heure et 72 heures, de préférence entre 3 et 24 heures, de préférence encore entre 3 heures et 10 heures.

Lors de l'étape de détection des compartiments comprenant au moins un microorganisme en division ou ayant une activité métabolique, chaque goutte 16 est placée dans la zone de mesure 32. La mesure comprend, par exemple, la mesure d'un signal de fluorescence indicatif de la croissance d'un microorganisme 2 dans la goutte 16.

Cette opération est par exemple répétée lors de l'étape de mesure d'un paramètre indicatif de la croissance d'un microorganisme 2 dans la goutte 16.

Selon un mode de réalisation particulier, la goutte 16 est placée dans la zone de mesure 32 avant l'étape d'incubation.

Avantageusement, le procédé comprend en outre une étape d'analyse des mesures effectuées pour la goutte 16, la mise en circulation de la goutte 16 vers la zone de préparation 37 pour la détermination d'un paramètre fonctionnel, la poursuite de l'incubation, la récupération de la goutte 16 ou l'évacuation de la goutte 16 dépendant du résultat de l'analyse. Cette étape est mise en oeuvre par l'unité centrale 24.

Par exemple, dès la détection de la présence d'un microorganisme dans une goutte 16, la goutte 16 est mise en circulation vers la zone de préparation 37 pour la détermination d'un paramètre fonctionnel, ou la goutte 16 est récupérée.

Avantageusement, la vitesse de croissance est déterminée par l'unité centrale 24. Après un ralentissement de la croissance du microorganisme, la goutte 16 est mise en circulation vers la zone de préparation 37 pour la détermination d'un paramètre fonctionnel, ou la goutte 16 est récupérée.

Par exemple, lorsqu'il est détecté dans la goutte 16 qu'une molécule est produite par un microorganisme 2 ou une population de microorganismes, la goutte 16 est mise en circulation vers la zone de préparation 37 pour la détermination d'un paramètre fonctionnel ou la goutte 16 est récupérée dans le récipient de récupération 80.

Si la mesure correspond à un critère de sélection de l'utilisateur par exemple lorsqu'il est mesuré que le microorganisme est encore en phase de croissance exponentielle, la goutte 16 est remise dans la zone d'incubation 30 par le dispositif de mise en circulation 18. Par exemple, il s'agit de la zone d'incubation 30 disposée en amont du dispositif d'identification 8. Selon un autre exemple, il s'agit d'une nouvelle zone d'incubation disposée en aval du dispositif d'identification 8.

Le critère est, par exemple, une quantité de biomasse finale ou une vitesse de croissance. Par exemple, lorsqu'il est détecté que la goutte 16 ne comprend pas de microorganismes, la goutte 16 est évacuée. De même, si la mesure ne correspond pas aux critères de sélection de l'utilisateur la goutte 16 est évacuée. En variante, au moins une goutte 16 ne comprenant pas de microorganismes est récupérée pour une caractérisation hors du système 1 ou mise en circulation vers la zone de préparation 37 pour la détermination d'un paramètre fonctionnel. Une telle goutte 16 sert de témoin négatif pour la caractérisation et/ou la détermination d'un paramètre fonctionnel.

De préférence, le critère est une quantité de biomasse finale suffisante pour mettre en œuvre l'étape de détermination d'au moins un paramètre fonctionnel relatif à un microorganisme 2. La goutte 16 est remise dans la zone d'incubation 30 par le dispositif de mise en circulation 18 jusqu'à atteindre la quantité de microorganismes 2 désirée.

Par exemple, l'incubation est prolongée jusqu'à ce que les gouttes 16 identifiées comme contenant au moins un microorganisme 2 vivant contiennent en cumulé au moins 10² cellules, au moins 10³ cellules, au moins 10⁴ cellules, au moins 10⁵ cellules ou encore au moins 10⁶ cellules.

Par exemple, le paramètre fonctionnel est une sensibilité à un antibiotique.

Selon cet exemple, le critère est une quantité de microorganismes supérieure ou égale à 5.10⁵ cellules de microorganismes par goutte, ou cumulées sur un nombre de gouttes compris entre 1 et 50. Cette quantité permet de préparer entre 30 et 1000 compartiments avec 5.10⁵ cellules par millilitre pour réaliser un antibiogramme.

Par exemple, au total l'incubation est prolongée pendant une durée comprise entre 30 minutes et 5 heures pour des microorganismes à croissance rapide, et jusqu'à 12h pour des microorganismes à croissance lente.

Selon cet exemple, la goutte 16 est ensuite mise en circulation vers la zone de préparation 37 des compartiments 6' pour la détermination d'un paramètre fonctionnel.

Par exemple, avant d'être récupérée, la goutte 16 positive subit une addition de réactifs. Le dispositif 79 d'addition d'au moins un réactif inocule au moins un réactif dans la goutte 16. Le réactif est par exemple choisi parmi de la matrice pour la spectrométrie de masse MALDI, des enzymes, de l'ADN et de l'ARN.

La goutte 16 est récupérée dans le dispositif de récupération 26.

Par exemple, les microorganismes 2 issus des gouttes 16 récupérées et utilisées pour préparer de nouveaux compartiments 6' sont resuspendus dans un ou une pluralité de réservoirs de microorganismes 86 dans un milieu de culture et dilués pour atteindre une densité de cellules contrôlée par une mesure de densité optique.

Le procédé comprend avantageusement en outre la génération d'un train 14' de gouttes 16' ordonnées dans le fluide porteur 40. Le train 14' de gouttes 16' est généré par le dispositif de préparation 11 des compartiments 6'.

Dans un exemple, entre 30 gouttes 16' et 1000 gouttes 16' sont générées pour former un ou plusieurs trains de gouttes.

Pour l'étape de détermination d'au moins un paramètre fonctionnel relatif à un microorganisme 2, les gouttes 16' sont par exemple inoculées avec un antibiotique.

Le dispositif d'inoculation inocule chaque goutte 16' ou plusieurs gouttes 16' dans des compartiments contenant un antibiotique issu d'un réservoir.

Les concentrations en antibiotique sont par exemple comprises entre 1 µg/L et 500 mg/L.

De préférence, le dispositif d'inoculation inocule des concentrations différentes d'un même antibiotique dans chaque goutte 16', et/ou un antibiotique différent dans chaque goutte 16'. Le dispositif d'inoculation inocule et prépare chaque goutte 16' de telle façon que le volume de chaque goutte 16' inoculée mise en circulation vers la zone de détermination d'un paramètre fonctionnel 33 est inférieur à 10µL, ou encore inférieur à 1µL.

Les gouttes 16' inoculées sont mises en circulation vers la zone d'incubation 30'. La zone d'incubation 30' du tube 10 est maintenue à une température de 37°C.

L'incubation est effectuée dans la zone d'incubation 30' du tube 10.

L'incubation est effectuée par phases successives, une mesure est effectuée à la fin de chaque phase d'incubation.

Avantageusement, chaque phase d'incubation dure 30 minutes.

Par exemple, au total l'incubation dure entre 1 heure et 24 heures, ou encore entre 3 heures et 10 heures.

Les gouttes 16' sont ensuite mises en circulation vers la zone de détermination d'un paramètre fonctionnel 33.

La détermination du paramètre fonctionnel comprend par exemple, la mesure d'un signal de fluorescence indicatif de la croissance d'un microorganisme en présence d'antibiotique dans les gouttes 16'.

Avantageusement, le procédé comprend en outre une étape d'analyse des mesures effectuées pour la goutte 16, 16', et de génération de rapports de résultats de cette analyse. Cette étape est mise en œuvre par l'unité centrale 24. Les résultats sont par exemple affichés ou imprimés par le dispositif d'affichage ou le dispositif d'impression du système 1.

Par exemple, avant ou après le transfert vers la zone de détermination d'un paramètre fonctionnel 33, et/ou en fin d'analyse, les gouttes 16, 16' sont récupérées dans le récipient de récupération 80. Chaque goutte 16, 16' sélectionnée est récupérée dans un réceptacle 82.

Avantageusement, le procédé comprend en outre une étape de récupération d'au moins une partie des compartiments 6 comprenant au moins un microorganisme 2 hors du système automatisé 1 d'isolement et d'analyse de microorganismes.

Par exemple, les gouttes 16, 16' sélectionnées par l'unité centrale 24 sont récupérées dans le récipient de récupération 80. Chaque goutte 16, 16' sélectionnée est récupérée dans un réceptacle 82'. Les gouttes 16, 16' sélectionnées sont par exemple utilisées hors du système 1 pour caractériser les microorganismes 2 qu'elles contiennent. La caractérisation est, par exemple, une identification des microorganismes présents ou une caractérisation Gram+/Gram -. Par exemple, les microorganismes 2 sont caractérisés par spectrométrie de masse MALDI-TOF, ou par l'utilisation de galeries API. Les gouttes16, 16' ainsi récupérées sont concentrées et permettent à un utilisateur de réaliser les analyses de son choix hors du système 1.

Avantageusement, les gouttes récupérées sont préparées automatiquement dans le système 1 sur le support adapté à la technique MALDI-TOF pour l'identification des espèces microbiennes et le support ainsi préparé est transféré automatiquement par un convoyeur dans un spectromètre de masse adapté pour réaliser la mesure.

En variante, le paramètre fonctionnel est une quantité d'azote et de phosphate métabolisés par le microorganisme, une quantité de toxines produites, ou une activité enzymatique.

En variante, les gouttes 16, 16' sélectionnées par l'unité centrale 24 sont groupées, manipulées et éventuellement redistribuées. Cela permet de garantir que l'ensemble des résultats obtenus dans le système 1 et hors du système 1 puisse être considéré comme descriptif d'un seul même échantillon 4 ou au moins de la même population de microorganismes 2 issue de l'échantillon 4 de départ.

En variante, la détermination du paramètre fonctionnel est mise en œuvre dans une cassette placée à une sortie du tube et pourvue d'une pluralité de réservoirs comprenant chacun un antibiotique et un dispositif d'inoculation propre à inoculer les gouttes sélectionnées dans les réservoirs. Chaque réservoir est un compartiment isolé des autres compartiments et de l'atmosphère ambiante. Par exemple, la cassette comprend entre 30 et 1000 réservoirs. Chaque réservoir est configuré pour comprendre en fin de préparation une concentration en antibiotique comprise entre 1 µg/L et 500 mg/L. Par exemple, certains réservoirs ne contiennent pas d'antibiotique.

Avantageusement, chaque réservoir comprend un indicateur de croissance de microorganismes.

Le dispositif de fermeture est fermé, il recouvre les réservoirs. Cela permet de limiter l'évaporation ou la dégradation des antibiotiques qu'ils contiennent.

Lors de l'inoculation de gouttes dans les réservoirs, l'unité centrale commande l'ouverture du dispositif de fermeture.

Le dispositif de déplacement de la cassette par rapport à la sortie du tube déplace la cassette de sorte que les gouttes à analyser soient utilisées pour inoculer les réservoirs ou les compartiments qui comprennent un antibiotique.

Lorsque l'inoculation est terminée, l'unité centrale commande la fermeture du dispositif de fermeture. La cassette est incubée pendant une durée déterminée, par exemple comprise entre 1 heures et 24 heures, ou encore entre 3 heures et 10 heures

La détermination du paramètre fonctionnel comprend par exemple, la mesure d'un signal de fluorescence indicatif de la croissance d'un microorganisme en présence d'antibiotique dans les réservoirs.

L'invention permet ainsi de réduire la durée d'isolement et d'analyse de microorganismes, et de limiter la survenue de contaminations en intégrant dans un même système automatisé les étapes de fragmentation, d'incubation, de détection et de détermination d'au moins un paramètre fonctionnel relatif à un microorganisme.

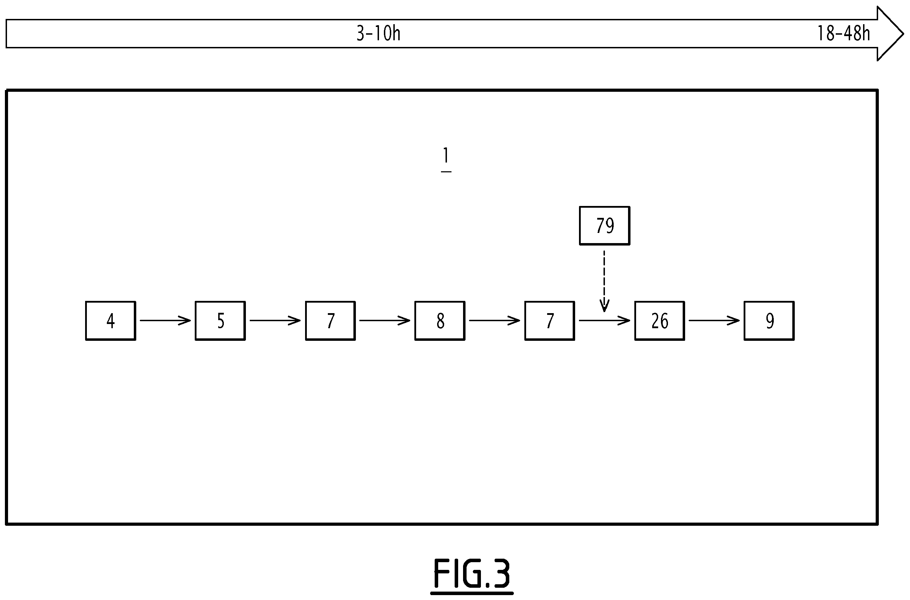

Comme illustré sur la figure 3, la durée cumulée des étapes de prélèvement, de fragmentation, d'incubation et de détection est inférieure à 48h, ou encore inférieure à 24h ou encore inférieure à 12h.

La présente invention sera illustrée plus en détail par l'exemple ci-dessous.

### Exemple :

Cet exemple démontre la détection et l'isolement des bactéries par la culture après compartimentation dans des gouttes et la mesure de Concentration Minimale Inhibitrice sur les gouttes isolées. Pour imiter les échantillons de patients pour l'infection du sang, la souche d'*Escherichia coli* (E. coli) K12 MC4100 a été diluée dans 20 mL de milieu Luria Broth pour atteindre moins de 5 bactéries par millilitre. Cette densité cellulaire initiale très faible correspond aux densités attendues dans les échantillons sanguins positifs. Les tests ont été effectués avec l'instrument Azur commercialisé par MilliDrop. L'instrument Azur commercialisé par MilliDrop permet de :
- préparer jusqu'à 1000 gouttes dont le volume est compris entre 400 nL et 1 µL à partir de 1 à 94 échantillons dont le volume est compris entre 30 µL et 20 mL,
- incuber les gouttes en mesurant un signal de fluorescence dans chaque goutte toutes les 30 min,
- trier les gouttes, après l'incubation et la mesure, pour ne conserver parmi les 1000 gouttes que celles choisies d'après le signal mesuré pendant l'incubation.

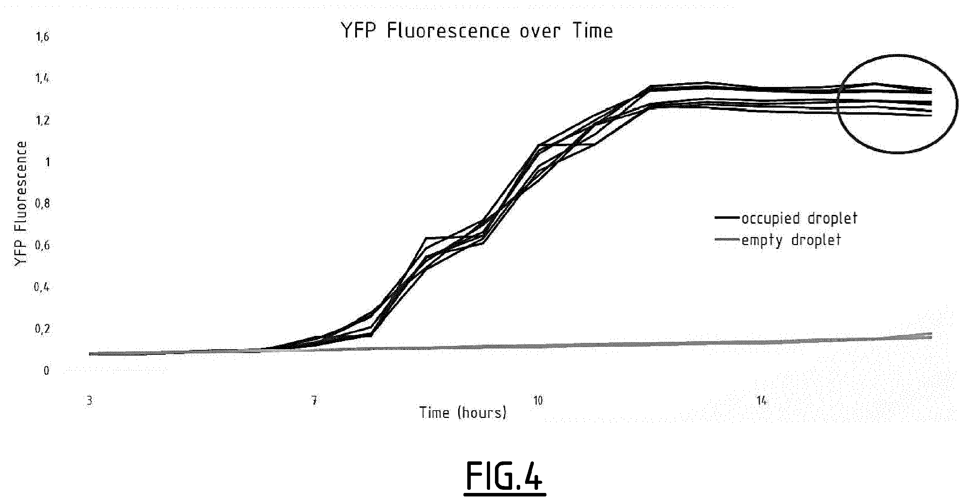

Cette méthode a permis de détecter les gouttes contenant des bactéries en croissance dès 8 heures après la préparation de l'échantillon (figure 4). Les gouttes contenant des bactéries en croissance sont appelées gouttes positives. Après une telle détection de gouttes positives, l'incubation est prolongée pendant 7 heures afin d'accumuler suffisamment de biomasse pour inoculer les tests de sensibilité aux antibiotiques (AST). Ensuite, les gouttes positives ont été collectées dans une plaques 96 puits en utilisant la fonctionnalité de tri des gouttes de l'instrument commercial Azur.

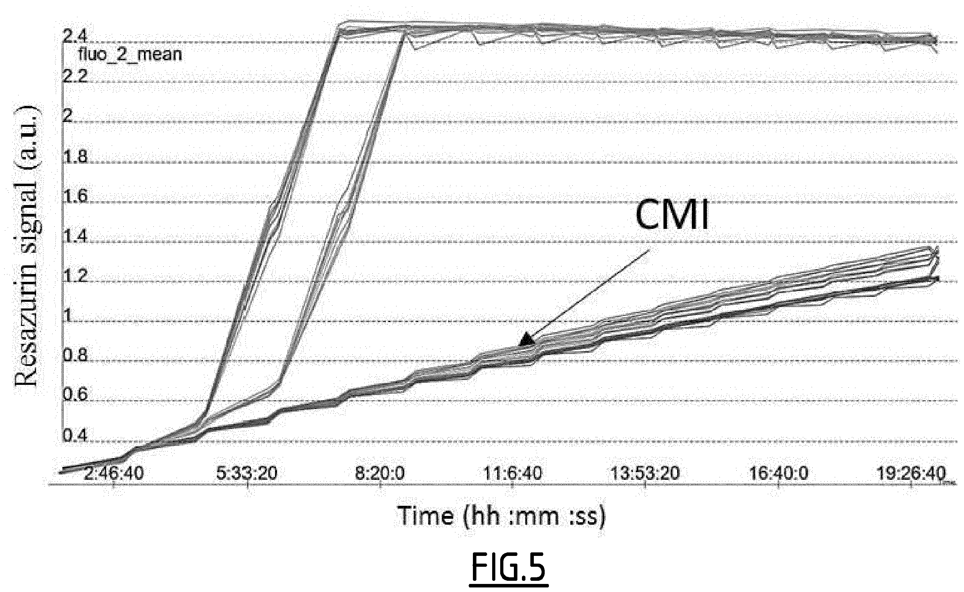

Une seule goutte positive collectée a ensuite été utilisée pour inoculer toutes concentrations d'antibiotiques préparée pour la détermination de la CMI. Les inventeurs ont testé 10 concentrations pour 3 antibiotiques différents. Chacune de ces 30 conditions a été reproduite dans 30 gouttes, ce qui représente 900 gouttes au total. Les échantillons ont été préparés pour atteindre un inoculum de 100 cellules par gouttes. Ensuite, l'incubation et la mesure des gouttes durent 8 heures pour déterminer les CMI (figure 5).

Il a été démontré que le temps d'incubation requis entre la réception de l'échantillon et l'AST était inférieur à 24 heures pour des souches infectieuses dont la vitesse de croissance est comparable à *Escherichia coli.* L'ensemble du procédé a nécessité 26h depuis la préparation de l'échantillon initial à l'obtention des résultats de CMI.

## Revendications

1. Procédé d'isolement et d'analyse de microorganismes (2) contenus dans un échantillon (4) comprenant les étapes suivantes :
- prélèvement d'un volume déterminé dans un échantillon (4), le volume déterminé représentant tout ou partie de cet échantillon (4), susceptible de contenir au moins un microorganisme (2),
- fragmentation du volume prélevé en une pluralité de compartiments (6) comprenant un milieu de culture (3), le volume de chaque compartiment (6) étant inférieur à 10 µL, chaque compartiment (6) étant isolé des autres compartiments (6) et dépourvu d'interface avec l'atmosphère ambiante,
- incubation de l'au moins un microorganisme (2) dans les compartiments (6) pendant une durée déterminée,
- avant, ou pendant l'incubation, détection des compartiments (6) comprenant au moins un microorganisme (2),
- prolongation de l'incubation après la détection des compartiments comprenant au moins un microorganisme (2) jusqu'à détecter une quantité de microorganisme (2) définie,
- récupération du contenu des compartiments (6) détectés comprenant au moins un microorganisme (2) dans des réceptacles (82) pour un usage ultérieur,
- détermination d'au moins un paramètre fonctionnel relatif à un microorganisme (2) dans les compartiments (6, 6') comprenant au moins un microorganisme (2),
dans lequel les étapes de fragmentation, d'incubation, de détection et de détermination d'au moins un paramètre fonctionnel sont intégrées dans un système automatisé (1) d'isolement et d'analyse de microorganismes (2).

2. Procédé d'isolement et d'analyse de microorganismes selon la revendication 1, dans lequel le contenu des compartiments (6), dans lesquels au moins un microorganisme (2) a été détecté, est récupéré dans des réceptacles (82) tandis que les autres compartiments (6) sont éliminés.

3. Procédé d'isolement et d'analyse de microorganismes selon la revendication 1 ou 2 dans lequel l'échantillon (4) est un échantillon de sang.

4. Procédé d'isolement et d'analyse de microorganismes selon l'une quelconque des revendications 1 à 3, dans lequel chaque compartiment (6) est une goutte (16).

5. Procédé d'isolement et d'analyse de microorganismes selon l'une quelconque des revendications 1 à 4, dans lequel le paramètre fonctionnel relatif à un microorganisme (2) est une sensibilité à un antibiotique.

6. Procédé d'isolement et d'analyse de microorganismes selon l'une quelconque des revendications 1 à 5, comprenant en outre, après l'étape de détection, une étape de récupération d'au moins une partie des compartiments (6) comprenant au moins un microorganisme (2) hors du système automatisé (1) d'isolement et d'analyse de microorganismes (2).

7. Procédé d'isolement et d'analyse de microorganismes selon l'une quelconque des revendications 1 à 6, comprenant en outre une étape de mesure d'un paramètre indicatif de la croissance d'un microorganisme (2) dans les compartiments (6) à différents instants.

8. Procédé d'isolement et d'analyse de microorganismes selon l'une quelconque des revendications 1 à 7, dans lequel la durée de l'étape d'incubation de l'au moins un microorganisme (2) dans les compartiments (6) est supérieure à 1 heure.

9. Procédé d'isolement et d'analyse de microorganismes selon l'une quelconque des revendications 1 à 8, comprenant en outre une étape de préparation des compartiments (6) pour la détermination de l'au moins un paramètre fonctionnel.

10. Procédé d'isolement et d'analyse de microorganismes selon l'une quelconque des revendications 4 à 9, dans lequel l'incubation est prolongée jusqu'à ce que les gouttes (16) identifiées comme contenant au moins un microorganisme (2) vivant contiennent en cumulé au moins 10³ cellules., et dans lequel la durée de la prolongation de l'incubation est par exemple au moins égale à 30 minutes.

11. Système automatisé (1) d'isolement et d'analyse de microorganismes (2) contenus dans un échantillon (4), le système comprenant :
- un dispositif de fragmentation (5) d'un volume déterminé d'un échantillon (4) susceptible de contenir au moins un microorganisme (2) en une pluralité de compartiments (6) dont le volume est inférieur à 10 µL, chaque compartiment (6) étant isolé des autres compartiments (6) et dépourvu d'interface avec l'atmosphère ambiante,
- un dispositif d'incubation (7) de l'au moins un microorganisme (2) dans les compartiments (6) pendant une durée déterminée,
- un dispositif de détection (8) des compartiments (6) comprenant au moins un microorganisme (2), et
- un dispositif de détermination (9) d'au moins un paramètre fonctionnel, propre à déterminer au moins un paramètre fonctionnel relatif à un microorganisme (2) dans les compartiments (6) comprenant au moins un microorganisme (2),
**caractérisé par** une unité centrale (24) de commande propre à piloter le dispositif d'incubation (7) et le dispositif de détection (8) pour réaliser :
- une incubation de l'au moins un microorganisme (2) dans les compartiments (6) pendant une durée déterminée,
- une détection des compartiments (6) comprenant au moins un microorganisme (2) avant ou après l'incubation, et
- une prolongation de l'incubation.

12. Système automatisé (1) d'isolement et d'analyse de microorganismes (2) contenus dans un échantillon (4) selon la revendication 11, le système (1) contenant dans le dispositif d'incubation (7), dans le dispositif de détection (8) ou dans le dispositif de détermination (9) d'au moins un paramètre fonctionnel, une pluralité de compartiments (6) dont le volume est inférieur à 10 µL.

13. Système automatisé (1) d'isolement et d'analyse de microorganismes (2) contenus dans un échantillon (4) selon la revendication 11 ou 12, dans lequel le dispositif de détermination (9) d'au moins un paramètre fonctionnel, propre à déterminer au moins un paramètre fonctionnel relatif à un microorganisme (2) dans les compartiments (6) comprenant au moins un microorganisme (2), est propre à mettre en œuvre un test de sensibilité à au moins un antibiotique dans les compartiments (6).

14. Système automatisé (1) d'isolement et d'analyse de microorganismes (2) contenus dans un échantillon (4) selon l'une quelconque des revendications 11 à 13, comprenant:
- un tube (10),
- un module de génération (12) d'un train (14) de gouttes (16) ordonnées dans un fluide porteur (40), chaque compartiment (6) correspondant à une goutte (16), le module de génération (12) d'un train (14) de gouttes (16) comprenant le dispositif de fragmentation (5), et
- un dispositif de mise en circulation (18) du train (14) de gouttes (16) dans le tube (10), propre à mettre en circulation le train (14) de gouttes (16) entre le dispositif de fragmentation (5), le dispositif d'incubation (7), le dispositif de détection (8) et le dispositif de détermination (9) d'au moins un paramètre fonctionnel.

15. Système automatisé (1) d'isolement et d'analyse de microorganismes (2) contenus dans un échantillon (4) selon la revendication 14, dans lequel le dispositif d'incubation (7) comprend au moins un dispositif de stockage des gouttes (16) propre à être régulé en température.

## Patentansprüche

1. Verfahren zur Isolierung und Analyse von Mikroorganismen (2), die in einer Probe (4) enthalten sind, die folgenden Schritte umfassend:
- Entnehmen eines bestimmten Volumens aus einer Probe (4), wobei das bestimmte Volumen die Gesamtheit oder einen Teil dieser Probe (4) darstellt, die geeignet ist, mindestens einen Mikroorganismus (2) zu enthalten,
- Fragmentieren des entnommenen Volumens in eine Vielzahl von Kompartimenten (6), die ein Nährmedium (3) enthalten, wobei das Volumen jedes Kompartiments (6) weniger als 10 µL beträgt und jedes Kompartiment (6) von den anderen Kompartimenten (6) isoliert ist und keine Grenzfläche mit der Umgebungsatmosphäre aufweist,
- Inkubieren des mindestens einen Mikroorganismus (2) in den Kompartimenten (6) während einer bestimmte Zeitdauer,
- vor oder während des Inkubierens Erfassen von Kompartimenten (6), die mindestens einen Mikroorganismus (2) enthalten,
- Verlängern der Inkubation nach dem Erfassen der mindestens einen Mikroorganismus (2) enthaltenden Kompartimente, bis eine definierte Menge des Mikroorganismus (2) nachgewiesen ist,
- Rückgewinnen des Inhalts der erfassten Kompartimente (6), die mindestens einen Mikroorganismus (2) enthalten, in Behältern (82) zur weiteren Verwendung,
- Bestimmen mindestens eines funktionellen Parameters, der sich auf einen Mikroorganismus (2) in den Kompartimenten (6, 6'), die mindestens einen Mikroorganismus (2) enthalten, bezieht,
bei dem die Schritte des Fragmentierens, Inkubierens, Erfassens und Bestimmens mindestens eines funktionellen Parameters in ein automatisiertes System (1) zur Isolierung und Analyse von Mikroorganismen (2) integriert sind.

2. Verfahren zur Isolierung und Analyse von Mikroorganismen nach Anspruch 1, bei dem der Inhalt der Kompartimente (6), in denen mindestens ein Mikroorganismus (2) nachgewiesen wurde, in Behältern (82) aufgefangen wird, während die anderen Kompartimente (6) entfernt werden.

3. Verfahren zur Isolierung und Analyse von Mikroorganismen nach Anspruch 1 oder 2, bei dem die Probe (4) eine Blutprobe ist.

4. Verfahren zur Isolierung und Analyse von Mikroorganismen nach einem beliebigen der Ansprüche 1 bis 3, bei dem jedes Kompartiment (6) ein Tropfen (16) ist.

5. Verfahren zur Isolierung und Analyse von Mikroorganismen nach einem beliebigen der Ansprüche 1 bis 4, bei dem der funktionelle Parameter, der sich auf einen Mikroorganismus (2) bezieht, eine Antibiotika-Empfindlichkeit ist.

6. Verfahren zur Isolierung und Analyse von Mikroorganismen nach einem beliebigen der Ansprüche 1 bis 5, umfassend nach dem Schritt des Erfassens außerdem einen Schritt des Wiedergewinnens mindestens eines Teils der Kompartimente (6), die mindestens einen Mikroorganismus (2) enthalten, aus dem automatisierten System (1) zum Isolieren und Analysieren von Mikroorganismen (2).

7. Verfahren zur Isolierung und Analyse von Mikroorganismen nach einem beliebigen der Ansprüche 1 bis 6, umfassend nach dem Schritt des Erfassens außerdem einen Schritt des Messens eines Parameters, der das Wachstum eines Mikroorganismus (2) in den Kompartimenten (6) angibt, zu verschiedenen Zeiten.

8. Verfahren zur Isolierung und Analyse von Mikroorganismen nach einem beliebigen der Ansprüche 1 bis 7, bei dem die Dauer des Schritts des Inkubierens des mindestens einen Mikroorganismus (2) in den Kompartimenten (6) mehr als 1 Stunde beträgt.

9. Verfahren zur Isolierung und Analyse von Mikroorganismen nach einem beliebigen der Ansprüche 1 bis 8, umfassend einen Schritt der Aufbereitung der Kompartimente (6) zum Bestimmen mindestens eines funktionellen Parameters.

10. Verfahren zur Isolierung und Analyse von Mikroorganismen nach einem beliebigen der Ansprüche 4 bis 9, bei dem die Inkubation so lange verlängert wird, bis die als mindestens einen lebenden Mikroorganismus (2) enthaltend identifizierten Tropfen (16) kumulativ mindestens 10³ Zellen enthalten, und bei dem die Dauer der Verlängerung der Inkubation beispielsweise mindestens 30 Minuten beträgt.

11. Automatisiertes System (1) zur Isolierung und Analyse von Mikroorganismen (2), die in einer Probe (4) enthalten sind, wobei das System umfasst:
- eine Vorrichtung (5) zum Fragmentieren eines gegebenen Volumens einer Probe (4), die geeignet ist, mindestens einen Mikroorganismus (2) zu enthalten, in eine Vielzahl von Kompartimenten (6), deren Volumen kleiner als 10 µL ist, wobei jedes Kompartiment (6) von den anderen Kompartimenten (6) isoliert ist und keine Grenzfläche mit der Umgebungsatmosphäre aufweist,
- eine Vorrichtung (7) zum Inkubieren des mindestens einen Mikroorganismus (2) in den Kompartimenten (6) während einer bestimmte Zeitdauer,
- eine Vorrichtung (8) zum Erfassen der Kompartimente (6), die mindestens einen Mikroorganismus (2) enthalten, und
- eine Vorrichtung (9) zum Bestimmen mindestens eines funktionellen Parameters, der sich auf einen Mikroorganismus (2) in den Kompartimenten (6), die mindestens einen Mikroorganismus (2) enthalten, bezieht,
**gekennzeichnet durch** eine zentrale Steuereinheit (24), die in der Lage ist, die Inkubationsvorrichtung (7) und die Erfassungsvorrichtung (8) zu steuern, zur Realisierung:
- einer Inkubation des mindestens einen Mikroorganismus (2) in den Kompartimenten (6) während einer bestimmte Zeitdauer,
- vor oder während der Inkubation eine Erfassung von Kompartimenten (6), die mindestens einen Mikroorganismus (2) enthalten, und
- eine Verlängerung der Inkubation.

12. Automatisiertes System (1) zur Isolierung und Analyse von in einer Probe (4) enthaltenen Mikroorganismen (2) nach Anspruch 11, wobei das System (1) in der Inkubationsvorrichtung (7), in der Erfassungsvorrichtung (8) oder in der Vorrichtung (9) zum Bestimmen mindestens eines funktionellen Parameters eine Mehrzahl von Kompartimenten (6) enthält, deren Volumen kleiner als 10 µL ist.

13. Automatisiertes System (1) zur Isolierung und Analyse von in einer Probe (4) enthaltenen Mikroorganismen (2) nach Anspruch 11 oder 12, bei dem die Vorrichtung (9) zum Bestimmen mindestens eines funktionellen Parameters, die geeignet ist, mindestens einen funktionellen Parameter in Bezug auf einen Mikroorganismus (2) in den mindestens einen Mikroorganismus (2) enthaltenden Kompartimenten (6) zu bestimmen, geeignet ist, einen Test auf Empfindlichkeit gegenüber mindestens einem Antibiotikum in den Kompartimenten (6) durchzuführen.

14. Automatisiertes System (1) zur Isolierung und Analyse von Mikroorganismen (2), die in einer Probe (4) enthalten sind, nach einem der Ansprüche 11 bis 13, umfassend:
- ein Rohr (10),
- ein Modul (12) zum Erzeugen einer Folge (14) von geordneten Tropfen (16) in einem Trägerfluid (40), wobei jedes Kompartiment (6) einem Tropfen (16) entspricht, wobei das Modul (12) zum Erzeugen einer Folge (14) von Tropfen (16) die Fragmentierungsvorrichtung (5) umfasst, und
- eine Vorrichtung (18) zum in Umlauf Bringen der Folge (14) von Tropfen (16) in dem Rohr (10), die geeignet ist, die Folge (14) von Tropfen (16) zwischen der Fragmentierungsvorrichtung (5), der Inkubationsvorrichtung (7), der Erfassungsvorrichtung (8) und der Vorrichtung (9) zum Bestimmen mindestens eines Funktionsparameters umlaufen zu lassen.

15. Automatisiertes System (1) zur Isolierung und Analyse von in einer Probe (4) enthaltenen Mikroorganismen (2) nach Anspruch 14, bei dem die Inkubationsvorrichtung (7) mindestens eine temperierbare Vorrichtung zum Speichern der Tropfen (16) umfasst.

## Claims

1. A process for the isolation and analysis of microorganisms (2) contained in a sample (4) comprising the following steps:
- collecting a determined volume in a sample (4), the determined volume representing all or part of this sample (4), likely to contain at least one microorganism (2),
- splitting the collected volume into a plurality of compartments (6) comprising a culture medium (3), the volume of each compartment (6) being smaller than 10 µL, each compartment (6) being isolated from the other compartments (6) and having no interface with the ambient atmosphere,
- incubating at least one microorganism (2) in the compartments (6) during a determined duration,
- before or during the incubation, detecting compartments (6) comprising at least one microorganism (2),
- extending the incubation after detecting compartments comprising at least one microorganism (2) until detecting a defined quantity of microorganism (2),
- recovering the content of the detected compartments (6) comprising at least one microorganism (2) in receptacles (82) for subsequent use,
- determining at least one functional parameter relative to a microorganism (2) in the compartments (6, 6') comprising at least one microorganism (2),
wherein the steps for splitting, incubation, detection and determining at least one functional parameter are integrated into an automated system (1) for isolating and analyzing microorganisms (2).

2. The process for isolating and analyzing microorganisms according to claim 1, wherein the content of the compartments (6), in which at least one microorganism (2) has been detected, is recovered in receptacles (82) while the other compartments (6) are eliminated.

3. The process for isolating and analyzing microorganisms according to claim 1 or 2, wherein the sample (4) is a blood sample.

4. The process for isolating and analyzing microorganisms according to any one of claims 1 to 3, wherein each compartment (6) is a drop (16).

5. The process for isolating and analyzing microorganisms according to any one of claims 1 to 4, wherein the functional parameter relative to a microorganism (2) is a sensitivity to an antibiotic.

6. The process for isolating and analyzing microorganisms according to any one of claims 1 to 5, wherein further comprising, after the detection step, a step for recovering at least part of the compartments (6) comprising at least one microorganism (2) outside the system (1) for automated isolation and analysis of microorganisms (2).

7. The process for isolating and analyzing microorganisms according to any one of claims 1 to 6, further comprising a step for measuring a parameter indicative of the growth of a microorganism (2) in the compartments (6) at different moments.

8. The process for isolating and analyzing microorganisms according to any one of claims 1 to 7, wherein the duration of the step for incubating at least one microorganism (2) in the compartments (6) is greater than 1 hour.

9. The process for isolating and analyzing microorganisms according to any one of claims 1 to 8, further comprising a step for preparing compartments (6) for the determination of at least one functional parameter.

10. The process for isolating and analyzing microorganisms according to any one of claims 4 to 9, wherein the incubation is prolonged until the drops (16) identified as containing at least one living microorganism (2) cumulatively contain at least 10³ cells, and wherein the duration of the prolongation of the incubation is for example at least equal to 30 minutes.

11. An automated system (1) for the isolation and analysis of microorganisms (2) contained in a sample (4), the system comprising:
- a device (5) for splitting a determined volume of a sample (4) likely to contain at least one microorganism (2) into a plurality of compartments (6) whose volume is smaller than 10 µL, each compartment (6) being isolated from the other compartments (6) and having no interface with the ambient atmosphere,
- a device (7) for incubating at least one microorganism (2) in the compartments (6) during a determined duration,
- a device (8) for detecting compartments (6) comprising at least one microorganism (2), and
- a device (9) for determining at least one functional parameter, capable of determining at least one functional parameter relative to a microorganism (2) in the compartments (6) comprising at least one microorganism (2),
**characterized by** a central control unit (24) capable of controlling the incubating device (7) and the detection device (8) in order to perform:
- an incubation of the at least one microorganism (2) in the compartments (6) during a determined duration,
- a detection of the compartments (6) comprising at least one microorganism (2) before or during the incubation, and
- a prolongation of the incubation.

12. The automated system (1) for isolating and analyzing microorganisms (2) contained in a sample (4) according to claim 11, the system (1) containing, in the incubation device (7), in the detection device (8) or in the device (9) for determining at least one functional parameter, a plurality of compartments (6) whose volume is smaller than 10 µL.

13. The automated system (1) for isolating and analyzing microorganisms (2) contained in a sample (4) according to claim 11 or 12, wherein the device (9) for determining at least one functional parameter, capable of determining at least one functional parameter relative to a microorganism (2) in the compartments (6) comprising at least one microorganism (2), is capable of carrying out a sensitivity test to at least one antibiotic in the compartments (6).

14. The automated system (1) for isolating and analyzing microorganisms (2) contained in a sample (4) according to any one of claims 11 to 13, comprising:
- a tube (10),
- a module (12) for generating a train (14) of sequenced drops (16) in a carrier fluid (40), each compartment (6) corresponding to a drop (16), the module (12) for generating a train (14) of drops (16) comprising the splitting device (5), and
- a device for circulating (18) the train (14) of drops (16) in the tube (10), capable of circulating the train (14) of drops (16) between the splitting device (5), the incubating device (7), the detection device (8) and the device (9) for determining at least one functional parameter.

15. The automated system (1) for isolating and analyzing microorganisms (2) contained in a sample (4) according to claim 14, wherein the incubating device (7) comprises at least one drop storage device (16) capable of being temperature-regulated.
